(19) Europäisches Patentamt
European Patent Office
Office européen des brevets

(11) Numéro de publication : **0 412 872 B1**

# (12) FASCICULE DE BREVET EUROPEEN

(45) Date de publication du fascicule du brevet : **16.12.92 Bulletin 92/51**

(51) Int. Cl.$^5$ : **B01J 31/14,** B01J 31/12, B01J 31/02, C07C 2/30, C07C 2/32

(21) Numéro de dépôt : **90402168.0**

(22) Date de dépôt : **27.07.90**

## (54) Nouvelle composition catalytique et sa mise en oeuvre pour l'oligomérisation des monooléfines.

(30) Priorité : **08.08.89 FR 8910758**

(43) Date de publication de la demande : **13.02.91 Bulletin 91/07**

(45) Mention de la délivrance du brevet : **16.12.92 Bulletin 92/51**

(84) Etats contractants désignés : **AT BE CH DE DK FR GB IT LI NL SE**

(56) Documents cités :
**EP-A- 0 008 304**
**EP-A- 0 012 685**
**EP-A- 0 231 748**
**US-A- 3 937 745**

(73) Titulaire : **INSTITUT FRANCAIS DU PETROLE**
**4, Avenue de Bois Préau**
**F-92502 Rueil-Malmaison (FR)**

(72) Inventeur : **Chauvin, Yves**
**64, avenue du Général Leclerc**
**F-78230 Le Pecq (FR)**
Inventeur : **Commereuc, Dominique**
**32, rue Abel Vacher**
**F-92190 Meudon (FR)**
Inventeur : **Forestière, Alain**
**1369, Chemin du Pelet**
**F-69390 Vernaison (FR)**
Inventeur : **Léger, Gérard**
**13 bis, rue Royet-Bat. C**
**F-69300 Caluire (FR)**

EP 0 412 872 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'objet de la présente invention est une nouvelle composition catalytique et sa mise en oeuvre pour l'oligomérisation, en particulier la dimérisation et la trimérisation, des monooléfines. Elle concerne plus précisément les combinaisons obtenues par mise en contact, dans un ordre quelconque, d'au moins un composé de nickel bivalent, avec au moins un halogènure d'hydrocarbylaluminium et au moins un composé époxy (appelé également oxiranne).

Il est bien connu de préparer des catalyseurs de dimérisation ou de codimérisation de monooléfines telles que l'éthylène, le propylène ou les n-butènes. De tels catalyseurs résultent par exemple : de l'interaction des halogénures de $\pi$-allyle nickel phosphine avec les acides de Lewis (FR-B-1 410 430), de l'interaction des halogénures de nickel phosphine avec les acides de Lewis (US-A-3 485 881) ou de l'interaction de certains carboxylates de nickel avec les halogénures d'hydrocarbylaluminium (US-A-3 321 546). D'autres catalyseurs mettent en oeuvre des composés zérovalents du nickel, qui sont cependant d'un emploi peu pratique en raison de leur instabilité et de leur coût élevé. On préfère en général utiliser des composés de nickel solubles dans les milieux hydrocarbonés.

La mise en oeuvre industrielle des compositions catalytiques décrites ci-dessus sur des coupes oléfiniques telles qu'elles sont issues des procédés pétrochimiques, comme le craquage catalytique ou le craquage à la vapeur, se heurte à des difficultés notamment liées aux impuretés contenues dans ces coupes.

Ces difficultés ont été en partie surmontées grâce à l'utilisation de formules catalytiques améliorées incorporant un composé de nickel bivalent, un halogénure d'hydrocarbylaluminium et un composé à caractère acide de Brönsted (US-A-4 283 305), ou un composé mixte du nickel en association avec un composé d'alkylaluminium (US-A-4 316 851, 4 366 087 et 4 398 049). Ces formules catalytiques améliorées incluent le plus souvent un acide halogénocarboxylique ou l'anion correspondant.

Il a été trouvé de façon inattendue que la mise en contact d'au moins un composé de nickel bivalent avec au moins un halogénure d'hydrocarbylaluminium et au moins un composé époxy conduit à une composition catalytique plus active que les formules de la technique antérieure, et présentant par ailleurs les mêmes propriétés vis-a-vis des impuretés éventuellement présentes dans les charges d'oligomérisation.

Les composés du nickel utilisables dans le cadre de l'invention sont tous les composés de nickel bivalent, de préférence solubles à plus de 0,1 g par litre en milieu hydrocarboné (par exemple dans l'heptane à +20 °C) et plus particulièrement dans les réactifs ou le milieu de réaction. Ils peuvent être par exemple constitués par l'acétylacétonate de nickel ou/et par des carboxylates de nickel de formule générale $(RCOO)_2$ Ni, où R est un reste hydrocarbyle, par exemple alkyle, cycloalkyle, alkényle, aryle, aralkyle ou alkaryle contenant jusqu'à 20 atomes de carbone, de préférence un reste hydrocarbyle de 5 à 20 atomes de carbone. Les deux radicaux R peuvent aussi former ensemble un reste alkylène de 6 à 18 atomes de carbone. Des exemples non-limitatifs de composés du nickel sont les sels de nickel bivalent suivants : octoate, éthyl-2 hexanoate, stéarate, oléate, naphténate, adipate.

Les halogénures d'hydrocarbylaluminium répondent de préférence à la formule générale $AlR'_xX_y$ où R'représente un groupement hydrocarboné monovalent contenant par exemple jusqu'à 12 atomes de carbone, tel que alkyle, aryle, aralkyle, alkaryle, cycloalkyle, X représente un halogène, choisi par exemple parmi le chlore, le brome et l'iode, de préférence X est un atome de chlore, x est compris entre 1 et 2, y est compris entre 1 et 2 avec $x + y = 3$, de préférence $x = 1$ et $y = 2$. Comme exemples de tels composés de formule $AlR'_xX_y$, on peut mentionner le sesquichlorure d'éthylaluminium, le dichloroétylaluminium, le dichloroisobutylaluminium et le chlorodiéthylaluminium.

Les composés époxy répondent de préférence à la formule générale suivante :

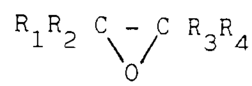

où $R_1$, $R_2$, $R_3$, $R_4$, identiques ou différents, sont en général choisis parmi le groupe formé par l'atome d'hydrogène et les groupements hydrocarbonés contenant par exemple jusqu'à 12 atomes de carbone, tels que alkyle, aryle ou cycloalkyle. Comme exemples de tels composés, on peut citer l'oxyde d'éthylène, l'oxyde de propylène, l'époxy-1,2 butane, l'époxy-styrène. $R_1$ et $R_4$ peuvent aussi former ensemble un reste alkylène de 4 à 10 atomes de carbone, $R_2$ et $R_3$ étant définis comme précédemment, comme par exemple dans l'époxy-1,2 cyclohexane.

Les trois composés entrant dans la composition catalytique selon l'invention peuvent être mélangés dans un ordre quelconque. On préfère cependant mélanger d'abord le composé de nickel bivalent avec le composé époxy, à une température généralement comprise entre -10 et +200°C, et de préférence, entre +20 et +100°C, éventuellement en présence d'un solvant hydrocarboné aliphatique ou aromatique ; le mélange ou la composition ainsi obtenu(e) est ensuite introduit(e), simultanément avec ou avant l'halogénure d'hydrocarbylaluminium, dans le milieu réactionnel d'oligomérisation.

Le rapport molaire du composé époxy au composé de nickel bivalent est en général compris entre 0,1:1 et 10:1 et, de préférence, entre 0,5:1 et 3:1. Le rapport molaire de l'halogénure d'hydrocarbylaluminium au composé de nickel bivalent est en général compris entre 1:1 et 50:1 et, de préférence, entre 2:1 et 20:1.

L'invention a aussi pour objet un procédé d'oligomérisation de monooléfines en présence de la composition catalytique définie ci-dessus, de préférence à une température comprise entre -20 et +100 °C, dans des conditions de pression telles que les réactifs soient maintenus au moins en majorité en phase liquide.

Les monooléfines susceptibles d'être dimérisées ou oligomérisées sont, par exemple, l'éthylène, le propylène, les n-butènes, les n-pentènes, purs ou sous forme de mélanges tels qu'issus des procédés de synthèse comme le craquage catalytique ou le craquage à la vapeur. Elle peuvent être cooligomérisées entre elles ou avec l'isobutène, par exemple l'éthylène avec le propylène et les n-butènes, le propylène avec les n-butènes, les n-butènes avec l'isobutène.

La concentration, exprimée en nickel, de la composition catalytique dans la phase liquide de réaction d'oligomérisation est normalement comprise entre 5 et 500 parties par million en poids.

Le procédé d'oligomérisation peut être mis en oeuvre notamment dans un réacteur à un ou plusieurs étages de réaction en série, la charge oléfinique et/ou les constituants du système catalytique étant introduits en continu, soit dans le premier étage, soit dans le premier et au moins un quelconque des étages. A la sortie du réacteur, le catalyseur peut être désactivé, par exemple à l'aide d'ammoniac et/ou d'une solution aqueuse de soude. Les oléfines non converties et les alcanes éventuellement présents sont ensuite séparés des oligomères par distillation.

Le procédé d'oligomérisation peut aussi être simplement conduit en "batch".

Les produits obtenus par le procédé d'oligomérisation selon l'invention trouvent leur application notamment comme composant de carburant pour automobile ou comme charge d'un procédé d'hydroformylation pour la synthèse d'aldéhydes et d'alcools.

Les exemples suivants illustrent l'invention sans toutefois en limiter la portée.

**EXEMPLE I**

Dans un autoclave en acier inoxydable de 300 ml de volume utile, on introduit 100 g d'une solution commerciale d'éthyl-2 hexanoate de nickel dans le white-spirit contenant 13 % en poids de nickel. On ferme l'autoclave que l'on purge sous vide, puis on le raccorde à une bouteille d'oxyde d'éthylène placée sur une balance. On chauffe l'autoclave à 70 °C et on déclenche l'agitation. Au bout de 35 minutes, on a absorbé 9,7 g d'oxyde d'éthylène, correspondant à un équivalent par atome-gramme de nickel. On isole l'autoclave et on le laisse refroidir. Puis on y introduit 100 ml d'heptane en agitant. On soutire la solution obtenue, qui est ensuite évaporée sous vide pour donner un liquide visqueux présentant une teneur en nickel de 13,45 % en poids.

**EXEMPLE 2**

Dans un autoclave en acier inoxydable de 250 ml de volume utile, préalablement séché et purgé avec de l'argon, on introduit 46 mg du mélange préparé dans l'exemple 1, en solution dans 5 ml d'heptane, soit 1,05 x $10^{-4}$ atome-gramme de nickel. Puis on injecte 100 g d'une solution à 11,5% en poids de propylène dans un mélange d'isohexènes issu d'une précédente opération de dimérisation du propylène. On règle la température à 40 °C au moyen d'une circulation d'eau dans la double enveloppe de l'autoclave, puis on injecte 0,2 g de dichloroéthylaluminium sous forme d'une solution commerciale à 50 % en poids dans l'hexane. On suit l'évolution de la réaction par des prélèvements dans l'autoclave échelonnés dans le temps. Au bout de 10 minutes de réaction, la conversion du propylène en oligomères est de 66,1 %.

**EXEMPLE 3** (comparatif)

Cet exemple illustre le résultat obtenu avec un catalyseur de l'art antérieur.

On utilise le même appareillage et le même mode opératoire que ceux décrits dans l'exemple 2, à ceci près qu'on introduit dans l'autoclave, à la place du mélange préparé dans l'exemple 1, 47,6 mg d'une solution commerciale d'éthyl-2 hexanoate de nickel dans le white-spirit contenant 13 % en poids de nickel, soit 1,05 x $10^{-4}$ atome-gramme de nickel. Dans les mêmes conditions que dans l'exemple 2 (en particulier une injection de dichloroéthylaluminium), la conversion du propylène en oligomères au bout de 10 minutes de réaction est de 24,1 %.

**EXEMPLE 4** (comparatif)

Cet exemple illustre le résultat obtenu avec une composition catalytique améliorée de l'art antérieur.

On utilise le même appareillage et le même mode opératoire que ceux décrits dans l'exemple 2, à ceci près qu'on introduit dans l'autoclave, à la place du mélange préparé dans l'exemple 1, 62 mg d'une solution d'éthyl-2 hexanoate de nickel dans le white-spirit contenant un équivalent d'acide trifluoroacétique par atome-gramme de nickel et ayant un titre de 10 % (en poids) en nickel, soit 1,05 x $10^{-4}$ atome-gramme de nickel. Dans les mêmes conditions que dans l'exemple 2 (en particulier une injection de dichloroéthylaluminium), la conversion du propylène en oligomères au bout de 10 minutes de réaction est de 58,9 %.

**EXEMPLE 5**

Dans le même appareillage que celui décrit dans l'exemple 2, on introduit 272 mg du mélange préparé dans l'exemple 1, en solution dans 5 ml d'heptane. Puis on injecte 118 g d'une coupe $C_4$ contenant en poids 14,7 % d'hydrocarbures saturés et 85,3 % de n-butènes. On règle également la température à 40 °C, puis on injecte 1,18 g de dichloroéthylaluminium sous forme d'une solution commerciale à 50 % en poids dans l'hexane. Après une heure de réaction, la conversion des n-butènes en oligomères est de 62,8 %.

**EXEMPLE 6**

On prépare un mélange entre l'éthyl-2 hexanoate de nickel et l'oxyde d'éthylène selon le mode opératoire décrit dans l'exemple 1, à ceci près qu'on laisse absorber 29,1 g d'oxyde d'éthylène pour 100 g de sel de nickel à 13 % en poids de nickel. Après traitement comme dans l'exemple 1, on obtient un mélange ayant une teneur en nickel de 11,47 % en poids.

**EXEMPLE 7**

On utilise le même appareillage et le même mode opératoire que ceux décrits dans l'exemple 5, à ceci près qu'on introduit dans l'autoclave, à la place du mélange préparé dans l'exemple 1, 319 mg du mélange préparé dans l'exemple 6, en solution dans 5 ml d'heptane. Dans les mêmes conditions que dans l'exemple 5 (en particulier une injection de dichloroéthylaluminium), la conversion des n-butènes en oligomères après une heure de réaction est de 60,1 %.

**EXEMPLE 8**

Dans un autoclave en acier inoxydable de 250 ml de volume utile, on introduit 100 g d'une solution commerciale d'éthyl-2 hexanoate de nickel dans le white-spirit contenant 13 % en poids de nickel, puis 100 ml d'heptane. Dans un sas relié à l'autoclave, on introduit 10 g d'oxyde d'éthylène, que l'on chasse ensuite dans l'autoclave par une surpression de 2 bars d'azote. On chauffe le mélange pendant 9 heures à 70 °C. Après refroidissement et évaporation sous vide de l'heptane, on obtient un liquide visqueux présentant une teneur en nickel de 13 % en poids.

**EXEMPLE 9**

Dans un réacteur muni d'une boucle de recirculation extérieure équipée d'un échangeur pour contrôler la température, dont le volume total est de 7,6 litres, on injecte en continu 1000 g/h d'une charge composée de 75 % de n-butènes, 6 % d'isobutène et 19 % de butanes. On injecte également en continu suivant deux flux séparés, d'une part 20 g/h d'une solution dans l'heptane du mélange préparé dans l'exemple 8 contenant 21,45 g de mélange étendu à 1000 g de solution et, d'autre part, 30 g/h d'une solution dans l'heptane contenant 6 % en poids de dichloroéthylaluminium. La température est fixée à 42 °C.

A la sortie du réacteur, l'effluent est détruit en continu par injections successives d'ammoniac gazeux et d'une solution aqueuse de soude à 15 % en poids.

L'analyse de l'effluent montre que la conversion des n-butènes est de 76,4 % et celle de l'isobutène de 72,2 %. Sa composition (en poids) est de 85,2 % d'octènes, 10,8 % de dodécènes, 2 % d'hexadécènes et 2 % d'oléfines supérieures à $C_{16}$. La sélectivité pour les dimères, calculée par rapport aux monomères transformés, est de 86,9 % pour les n-butènes et de 58,1 % pour l'isobutène.

**EXEMPLE 10** (comparatif)

Cet exemple illustre le résultat obtenu avec une composition catalytique de l'art antérieur.

On utilise le même appareillage en continu et les mêmes conditions opératoires que ceux décrits dans l'exemple 9, à ceci près que le flux de la solution du mélange préparé dans l'exemple 8 est remplacé par un flux de 20 g/h d'une solution dans l'heptane d'éthyl-2 hexanoate de nickel contenant un équivalent d'acide trifluoroacétique par atome-gramme de nickel et ayant un titre (en poids) de 0,28 % en nickel, ce qui correspond à la même quantité horaire de nickel que dans l'exemple 9.

L'analyse de l'effluent montre que la conversion des n-butènes est de 72,6 % et celle de l'isobutène de 90,9 %. Sa composition (en poids) est de 78,6 % d'octènes, 13,1 % de dodécènes, 4,3 % d'hexadécènes et 4 % d'oléfines supérieures à $C_{16}$. La sélectivité pour les dimères, calculée par rapport aux monomères transformés, est de 83,7 % pour les n-butènes et de 22,8 % pour l'isobutène.

## Revendications

1. Composition catalytique caractérisée en ce qu'elle est le produit résultant de la mise en contact, dans un ordre quelconque, d'au moins un composé de nickel bivalent avec au moins un halogénure d'hydrocarbylaluminium et au moins un composé époxy.

2. Composition catalytique selon la revendication 1 caractérisée en ce que le composé époxy répond à la formule générale suivante :

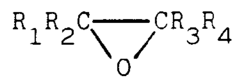

où $R_1$, $R_2$, $R_3$, $R_4$, identiques ou différents, sont choisis parmi le groupe formé par l'atome d'hydrogène et les groupements hydrocarbonés contenant jusqu'à 12 atomes de carbone ou $R_1$ et $R_4$ forment ensemble un reste alkylène de 4 à 10 atomes de carbone, $R_2$ et $R_3$, identiques ou différents, étant choisis parmi le groupe formé par l'atome d'hydrogène et les groupements hydrocarbonés contenant jusqu'à 12 atomes de carbone.

3. Composition catalytique selon la revendication 2 caractérisée en ce que le composé époxy est choisi dans le groupe formé par l'oxyde d'éthylène, l'oxyde de propylène, l'époxy-1,2 butane, l'époxystyrène et l'époxy-1,2 cyclohexane.

4. Composition catalytique selon l'une des revendications 1 à 3 caractérisée en ce que l'halogénure d'hydrocarbylaluminium répond à la formule générale $AlR'_xX_y$ où R′ représente un groupement hydrocarboné monovalent contenant jusqu'à 12 atomes de carbone, X représente un halogène choisi parmi le chlore, le brome et l'iode, x est compris entre 1 et 2 et y est compris entre 1 et 2, avec x + y = 3.

5. Composition catalytique selon la revendication 4 caractérisée en ce que, dans la formule générale de l'halogénure d'hydrocarbylaluminium, x est égal à 1, y est égal à 2 et X représente le chlore.

6. Composition catalytique selon l'une des revendications 1 à 5 caractérisée en ce que le composé de nickel bivalent est choisi parmi le groupe formé par l'acétylacétonate de nickel et les carboxylates de nickel de formule $(RCOO)_2Ni$ où R est un reste hydrocarbyle de 5 à 20 atomes de carbone ou les deux R forment ensemble un reste alkylène de 6 à 18 atomes de carbone.

7. Composition catalytique selon l'une des revendications 1 à 6 dans laquelle le rapport molaire de l'halogénure d'hydrocarbylaluminium au composé de nickel bivalent est compris entre 1:1 et 50:1 et le rapport molaire du composé époxy au composé de nickel bivalent est compris entre 0,1:1 et 10:1.

8. Composition catalytique selon l'une des revendications 1 à 6 dans laquelle le rapport molaire de l'halogénure d'hydrocarbylaluminium au composé de nickel bivalent est compris entre 2:1 et 20:1 et le rapport molaire du composé époxy au composé de nickel bivalent est compris entre 0,5:1 et 3:1.

9. Utilisation d'une composition catalytique selon l'une des revendications 1 à 8 dans un procédé d'oligomérisation de monooléfines.

**Claims**

1. A catalytic composition, characterised in that it is the product resulting from putting at least one divalent nickel compound into contact with at least one hydrocarbylaluminium halide and at least one epoxy compound, in any order.

2. The catalytic composition of Claim 1, characterised in that the epoxy compound is of the following general formula

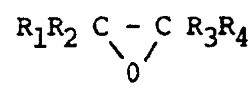

where $R_1$, $R_2$, $R_3$and $R_4$ are the same or different and are selected from the group formed by the hydrogen atom and hydrocarbon groups containing up to 12 carbon atomd, or $R_1$ and $R_4$ together form an alkylene radical with 4 to 10 carbon atoms, $R_2$ and $R_3$, which may be the same or different, being selected from the group formed by the hydrogen atom and hydrocarbon groups containing up to 12 carbon atoms.

3. The catalytic composition of Claim 2, characterised in that the epoxy compound is selected from the group formed by ethylene oxide, propylene oxide, 1,2-epoxy butane, epoxy styrene and 1,2-epoxy cyclohexane.

4. The catalytic composition of any of Claims 1 to 3 characterised in that the hydrocarbylaluminium halide is of the general formula $AlR'_xX_y$ where R′ represents a monovalent hydrocarbon group containing up to 12 carbon atoms X represents a halogen selected from chlorine, bromine and iodine, x is from 1 to 2 y is from 1 to 2, with x + y = 3.

5. The catalytic composition of Claim 4, characterised in that in the general formula for the hydrocarbylalyminium halide, x = 1, y = 2 and X represents chlorine.

6. The catalytic composition of any of Claims 1 to 5 characterised in that the divalent nickel compound is selected from the group formed by nickel acetylacetonate and nickel carboxylates of the formula $(RC00)_2Ni$ where R is a hydrocarbyl radical with 5 to 20 carbon atoms or the two R's together form an alkylene radical with 6 to 18 carbon atoms.

7. The catalytic composition of any of Claims 1 to 6, wherein the molar ratio of hydrocarbylaluminium halide to divalent nickel compound is from 1:1 to 50:1, and the molar ratio of epoxy compound to divalent nickel compound is from 0.1:1 to 10:1.

8. The catalytic composition of any of Claims 1 to 6, wherein the molar ratio of hydrocarbylaluminium halide to divalent nickel compound is from 2:1 to 20:1, and the molar ratio of epoxy compound to divalent nickel compound is from 0.5:1 to 3:1.

9. Use of the catalytic composition of any of Claims 1 to 8 in a process for oligomerising monoolefins.

## Patentansprüche

1. Katalytische Zusammensetzung, dadurch gekennzeichnet, daß sie das Produkt ist, das entsteht, wenn man in beliebiger Reihenfolge mindestens eine Verbindung von zweiwertigem Nickel mit mindestens einem kohlenwasserstoffsubstituierten Aluminiumhalogenid und mindestens einer Epoxiverbindung in Kontakt bringt.

2. Katalytische Zusammensetzung gemäß Anspruch 1, dadurch gekennzeichnet, daß die Epoxiverbindung der folgenden allgemeinen Formel entspricht:

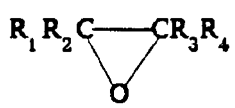

in der $R_1$, $R_2$, $R_3$ und $R_4$, identisch oder voneinander verschieden, aus der Gruppe gewählt werden, die vom Wasserstoffatom und den Kohlenwasserstoffgruppen mit bis zu 12 Kohlenstoffatomen gebildet wird oder $R_1$ und $R_4$ gemeinsam einen Alkylenrest mit 4 bis 10 Kohlenstoffatomen bilden, und $R_1$ und $R_3$, identisch oder voneinander verschieden, aus der Gruppe gewählt werden, die vom Wasserstoffatom und den Kohlenwasserstoffgruppen mit bis zu 12 Kohlenstoffatomen gebildet wird.

3. Katalytische Zusammensetzung gemäß Anspruch 2, dadurch gekennzeichnet, daß die Epoxiverbindung aus der Gruppe gewählt wird, die durch Ethylenoxid, Propylenoxid, Epoxi-1,2 butan, Epoxistyrol und Epoxi-1,2 cyclohexan gebildet wird.

4. Katalytische Zusammensetzung gemäß einem der Ansprüche 1 bis 3. dadurch gekennzeichnet, daß das kohlenwasserstoffsubstituierte Aluminiumhalogenid der allgemeinen Formel $AlR'_xX_y$ entspricht, wobei R′ einer monovalenten Kohlenwasserstoffgruppe entspricht, die bis zu 12 Kohlenstoffatome enthält und X ein Halogen darstellt, das unter Chlor. Brom und Jod gewählt wird, x ist zwischen 1 und 2 enthalten und y ist zwischen 1 und 2 enthalten , wobei gilt: x+y=3.

5. Katalytische Zusammensetzung gemäß Anspruch 4, dadurch gekennzeichnet, daß in der allgemeinen Formel des kohlenwasserstoffsubstituierten Aluminiumhalogenids x gleich 1 ist, y gleich 2 ist und X für Chlor steht.

6. Katalytische Zusammensetzung gemäß einem der Ansprüche 1 bis 5, dadurch gekennzeichnet, daß die Verbindung von zweiwertigem Nickel aus der Gruppe gewählt wird, die aus Nickelacetylacetonat und den Nickelcarboxylaten der Formel $(RCOO)_2Ni$ besteht, wobei R einen Kohlenwasserstoffrest mit zwischen 5 und 20 Kohlenstoffatomen darstellt oder die beiden Reste R zusammen einen Alkylenrest mit 6-18 Kohlenstoffatomen bilden.

7. Katalytische Zusammensetzung gemäß einem der Ansprüche 1 bis 6, in der das molare Verhältnis zwischen dem kohlenwasserstoffsubstituierten Aluminiumhalogenid und der Verbindung von zweiwertigem Nickel zwischen einschließlich 1:1 und 50:1 liegt und das molare Verhältnis der Epoxiverbindung und der Verbindung von zweiwertigem Nickel zwischen einschließlich 0.1:1 und 10:1 liegt.

8. Katalytische Verbindung gemäß einem der Ansprüche 1 bis 6, in der das molare Verhältnis des kohlenwasserstoffsubstituierten Aluminiumhalogenids und der Verbindung von zweiwertigem Nickel zwischen einschließlich 2:1 und 20:1 liegt und das molare Verhältnis zwischen der Epoxiverbindung und der Verbindung von zweiwertigem Nickel zwischen einschließlich 0.5:1 und 3:1 liegt.

9. Verwendung einer katalytischen Zusammensetzung gemäß einem der Ansprüche 1 bis 8 in einem Prozeß zur Oligomerisierung von Monoolefinen.